# EUROPÄISCHE PATENTSCHRIFT

(11) **EP 1 063 924 B1**
(45) Veröffentlichungstag und Bekanntmachung des Hinweises auf die Patenterteilung: **27.08.2003**
(21) Anmeldenummer: 99915625.0
(22) Anmeldetag: 16.03.1999
(51) Int. Cl.: A61B 17/17

(54) **VORRICHTUNG ZUM AUFFINDEN VON VERRIEGELUNGSBOHRUNGEN VON IMPLANTATEN**
DEVICE FOR LOCATING LOCKING HOLES OF IMPLANTS
DISPOSITIF PERMETTANT DE REPERER LES TROUS D'ANCRAGE DES IMPLANTS

(30) Priorität: 19.03.1998 DE 19812129
(43) Veröffentlichungstag der Anmeldung: 03.01.2001
(73) Patentinhaber: Wolter, Matthias, 20148 Hamburg (DE)
(72) Erfinder: Wolter, Matthias, 20148 Hamburg (DE)
(74) Vertreter: Siewers, Gescha, Dr.
(86) Internationale Anmeldenummer: EP9901719
(87) Internationale Veröffentlichungsnummer: WO99047052

(56) Entgegenhaltungen:
- WO-A-93/02626
- DE-A- 19 640 474
- US-A- 4 621 628
- US-A- 5 707 375

## Beschreibung

Die Erfindung betrifft eine Vorrichtung zum Auffinden von Verriegelungsbohrungen von Implantaten, insbesondere von distalen Verriegelungslöchern von Knochennägeln.

In der Chirurgie werden Implantate häufig mit quer zu erwartenden Druckoder Zugbelastung eingeschobenen Verriegelungsstiften befestigt. Insbesondere bei der Behandlung des Bruchs eines Röhrenknochens wird zur Stabilisierung des Knochens eine hohler oder kompakter Metallnagel als Implantat längs in den Markraum des Knochens intramedullär eingetrieben und mit quer dazu verlaufenden, stiftförmigen Elementen verriegelt. Durch diese Verriegelung wird die Fraktur in der korrekten Position fixiert und die richtige Länge des Knochens sichergestellt.

Ein erhebliches Problem bei dieser Technik ist jedoch, daß die im Knochennagel vorhandenen Querbohrungen zur Aufnahme der Verriegelungsstifte bei in den Knochen eingebrachtem Nagel von außen nicht sichtbar sind. Um dieses Problem zu lösen, sind in der Vergangenheit verschiedene Methoden entwickelt worden. Aus der US 5,127,913 ist beispielsweise ein Verfahren und eine Vorrichtung bekannt, bei der in der Verriegelungsbohrung des Implantats ein ein axialsymmetrisches Magnetfeld erzeugender Magnet angeordnet ist, in dessen Magnetfeld sich ein weiterer Magnet frei beweglich ausrichten kann. Der äußere Magnet wird in der Nähe des angenommenen Endes des Implantats im Magnetfeld des im Verriegelungsloch befindlichen Magneten bewegt. Er richtet sich so aus, daß seine Achse auf das Verriegelungsloch zeigt. Durch immer dichteres Heranführen an das Verriegelungsloch kann durch die Ausrichtung des Magneten ermittelt werden, in welcher Richtung der Knochen aufzubohren ist, um das Verriegelungsloch zu treffen. Problematisch bei dieser Vorgehensweise ist jedoch, daß die Symmetrieachse des axialsymmetrischen Magnetfeldes nur durch fortdauernde Bewegung des äußeren Magneten im Magnetfeld des Magneten im Verriegelungsloch zu erreichen ist. Befindet sich der Magnet außerhalb der Symmetrieachse, wird er sich bei Entfernung von der Bohrstelle im Magnetfeld drehen, wobei diese Drehung aufgrund des Feldlinienverlaufs um so stärker ist, je weiter er vom Verriegelungsloch entfernt wird. Der Präzision sind dabei Grenzen gesetzt, da das Magnetfeld mit zunehmender Entfernung vom erzeugenden Magneten immer schwächer wird. Die Sicherheit des Verfahrens ist also dadurch eingeschränkt, daß die Mechanik eine gewisse Trägheit aufweist und insbesondere in der Nähe der Symmetrieachse möglicherweise erst eine Drehung des Magneten wahrnehmbar wäre, wenn diese aufgrund der Schwergängigkeit der Mechanik und der abnehmenden Feldstärke nicht mehr erreicht wird. Es muß deshalb in Kauf genommen werden, daß möglicherweise nicht genau die Achse der Verriegelungsbohrung beim Aufbohren des Knochens getroffen wird.

US 5 707 375 beschreibt eine Vorrichtung zum Auffinden von Implantaten, bestehend aus einer frei im Magnetfeld eines sich im Verriegelungsloch des Implantates befindlichen Magneten beweglichen Magneteinrichtung.

WO-A-9302626 beschreibt eine Vorrichtung mit zwei Detektionsspulen, die auf einem gemeinsamen Rahmen angebracht sind. Die Detektionsspulen sind im Magnetfeld frei beweglich. Der gemeinsame Rahmen wird solange bewegt, bis ein an die Magnetspulen angeschlossener Detektor das maximale Signal anzeigt.

Es ist Aufgabe der vorliegenden Erfindung, eine verbesserte Meßvorrichtung vorzuschlagen. Insbesondere soll ein sicheres Auffinden des Verriegelungslochs und eine sichere Bestimmung der Achse gegeben sein. Abweichungen von der Achse sollen objektiv feststellbar sein.

Erfindungsgemäß wird eine Vorrichtung zum Auffinden von Verriegelungsbohrungen von Implantaten, insbesondere von distalen Verriegelungslöchern von Knochennägeln vorgeschlagen, bestehend aus einer sich frei im Magnetfeld eines im Verriegelungsloch des Implantats befindlichen Magneten beweglichen ersten Magneteinrichtung, die dadurch gekennzeichnet ist, daß eine zweite, von der ersten Magneteinrichtung beabstandet angeordnete, ebenfalls frei bewegliche Magneteinrichtung vorgesehen ist.

Die erfindungsgemäße Vorrichtung besteht im wesentlichen aus zwei Magneten, die sich frei im Magnetfeld eines Magneten bewegen können, der in das Verriegelungsloch des Implantats eingebracht ist. Aus dem Stand der Technik sind viele Beispiele bekannt, welcher Art die in dem Verriegelungsloch befindliche Magneteinrichtung sein kann. Allen bekannten Verfahren gemeinsam ist jedoch, daß es sich um eine Magneteinrichtung handelt, die axialsymmetrisches Magnetfeld erzeugt. Die beiden Magneteinrichtungen der erfindungsgemäßen Vorrichtung können z. B. übliche Stabmagnete sein. Sie können auf einer Halterung montiert sein, so daß sie sowohl frei drehbar, als auch bewegbar sind. Zur Bestimmung der Position und der Ausrichtung des Magneten im Verriegelungsloch wird die Vorrichtung so um das Verriegelungsloch herum angeordnet, bis sich beide Magneten fluchtend miteinander ausrichten. Ihre gemeinsame Achse entspricht dann der Achse des Verriegelungslochs.

Die erfindungsgemäße Vorrichtung läßt sich ausgesprochen einfach, ohne großen Aufwand und ohne empfindliche Geräte einsetzen. Bei der Verwendung von Stabmagneten bedingt sie nicht das Vorhandensein von Strom, so daß sie auch unter primitivsten Bedingungen zuverlässig arbeitet.

Eine erfindungsgemäße Weiterbildung besteht darin, daß wenigstens eine Magneteinrichtung aus einem Elektromagneten besteht. Auf diesem Wege kann zunächst diejenige Magneteinrichtung, die aus einem Permanentmagneten besteht, in herkömmlicher Art und Weise auf die Verriegelungsbohrung ausgerichtet werden. Die Feinjustierung kann dann unter Zuhilfenahme eines zuzuschaltenden Elektromagneten in Form der zweiten Magneteinrichtung erfolgen.

Selbstverständlich können auch beide Magneteinrichtungen Elektromagnete darstellen.

Weiterhin sollte mindestens eine Magneteinrichtung vorzugsweise kardanisch aufgehängt sein, in einer bevorzugten Ausführungsform sollten beide Magneteinrichtungen kardanisch ausgehängt sein.

Beide Magneteinrichtungen sollten darüber hinaus ausbalanciert sein, damit die Erdanziehungskraft nicht vom Magnetfeld und den darin auftretenden Kräften ausgeglichen werden muß.

Das Ausbalancieren einer oder beider Magneteinrichtungen kann durch ein Gegengewicht erzeugt werden, das ebenfalls magnetisch ist, so daß die im Magnetfeld des im Verriegelungsloch, befindlichen Magneten auftretenden Kräfte verstärkt werden.

In einer bevorzugten Ausführungsform der erfindungsgemäßen Vorrichtung sind beide Magneteinrichtungen auf einer Halterung derart justierbar angeordnet, daß ihr Abstand einstellbar ist. Weiterhin ist vorzugsweise eine optische und/oder akustische Anzeige vorgesehen, die auf eine vorgegebene Nullstellung einstellbar ist. Diese Ausgestaltung ermöglicht es, die beiden Magneten durch die gegenseitige Anziehung zunächst aufeinander auszurichten, so daß die von ihnen erzeugten Magnetfelder entlang einer Achse axialsymmetrisch verlaufen. Diese Einstellung ist die Nullstellung, auf die die optische und/oder akustische Anzeige einstellbar ist. Sodann kann die Magneteinrichtung in das Magnetfeld des im Verriegelungsloch befindlichen Magneten eingebracht werden. Die Vorrichtung muß dann solange im Magnetfeld bewegt werden, bis beide Magnete sich in der Nullstellung befinden. Sodann stimmt ihre Achse mit der Symmetrieachse des Magnetfeldes der Magneteinrichtung im Verriegelungsloch überein, die gleichzeitig die Achse des Verriegelungslochs darstellt.

Weiter vorzugsweise ist mindestens eine der beiden Magneteinrichtungen, am besten aber beide Magneteinrichtungen, arretierbar. Im Falle der Verwendung von Elektromagneten ist so möglich, zunächst einen Elektromagneten einzuschalten, die Lage des Verriegelungslochs in erster Näherung zu bestimmen, den Magneten in dieser Position zu arretieren und auszuschalten, mit der zweiten Magneteinrichtung in gleicher Weise fortzufahren, usw., bis die Justierung erreicht ist.

Im folgenden wird ein bevorzugtes Ausführungsbeispiel der erfindungsgemäßen Vorrichtung anhand der beigefügten Abbildung näher erläutert:

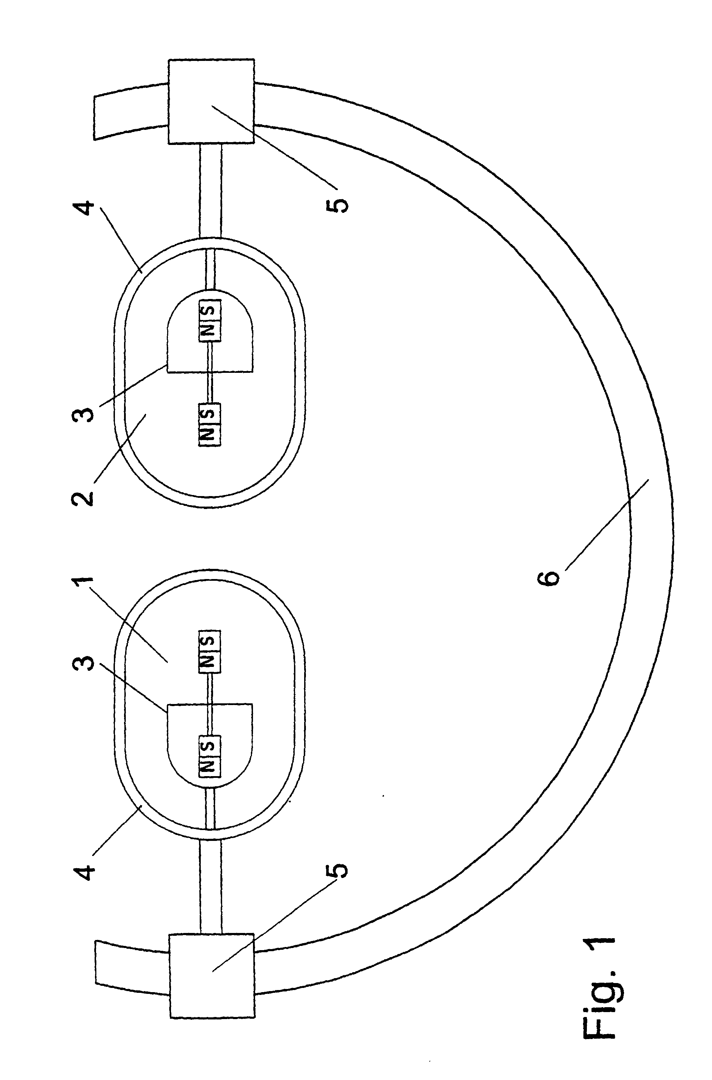

- **Fig. 1**: zeigt den Aufbau einer erfindungsgemäßen Vorrichtung zum Auffinden von Verriegelungsbohrungen in schematischer Darstellung.

Die erste Magneteinrichtung 1 und die zweite Magneteinrichtung 2 sind mit zweckentsprechenden justierbaren Haltevorrichtungen 5 auf einem halbkreisförmigen Halter 6 montiert. Sie sind an kardanischen Aufhängungen 3 frei ausbalanciert im Raum beweglich. Bei den Magneteinrichtungen 1, 2 handelt es sich um Stabmagnete mit axialsymmetrischem Magnetfeld, die paarweise angeordnet sind, um die gewünschte Balance herzustellen. Alternativ können selbstverständlich auch Elektromagnete verwendet werden.

Weiterhin ist jede Magneteinrichtung mit einer optischen Anzeige 4 versehen, an der ablesbar ist, ob die Magnete aufeinander ausgerichtet sind. Die Ausrichtung der Magnete aufeinander entspricht einer Nullstellung.

Die Haltevorrichtungen 5 sind entlang der halbkreisförmigen Halterung 6 verschiebbar und verstellbar, so daß der Abstand der Magneteinrichtungen 1 und 2 voneinander einstellbar ist und die Magneteinrichtungen aufeinander in Nullstellung gebracht werden können.

Zunächst wird der Abstand der beiden Magneteinrichtungen 1, 2 voneinander durch Verschieben und Einstellen der Haltevorrichtungen 5 auf dem halbkreisförmigen Halter 6 derart gewählt, daß das Körperteil des Patienten, in dem sich das Implantat befindet, ohne Schwierigkeiten zwischen die beiden Magneteinrichtungen eingeführt werden kann, und sich die Anzeigevorrichtungen in Nullstellung befinden.

Danach wird die Vorrichtung an den Patienten in der Nähe des Implantats herangeführt.

Beide Magneteinrichtungen 1 und 2 werden sich aus ihrer Nullage herausbewegen. Die erfindungsgemäße Vorrichtung wird dann solange im Magnetfeld des Magneten im Verriegelungsloch bewegt, bis sich beide Magnete wieder in ihrer Nullage befinden. Damit entspricht ihre Achse der ^{*} Achse des Verriegelungslochs. Die Nullage der Magnete kann entweder rein optisch durch eine Skala oder eine im Fall des Erreichens der Nullage beider Magnete aufleuchtenden Lampe oder durch ein akustisches Signal angezeigt werden.

Es kann darüber hinaus vorgesehen sein, daß die Magnete in bekannter Weise gegen eine Bohrvorrichtung austauschbar sind, die in die justierbaren Halterungen 5 einsetzbar ist.

## Patentansprüche

1. Vorrichtung zum Auffinden von Verriegelungsbohrungen von Implantaten, die eine sich frei im Magnetfeld eines im Verriegelungsloch des Implantats befindlichen Magneten bewegliche erste Magneteinrichtung (1) aufweist, **dadurch gekennzeichnet, daß** eine zweite, von der ersten Magneteinrichtung (1) beabstandet angeordnete, ebenfalls frei bewegliche Magneteinrichtung (2) vorgesehen ist, wobei sich die erste Magneteinrichtung (1) und die zweite Magneteinrichtung (2) frei im Magnetfeld eines in der Verriegelungsbohrung angebrachten Magneten bewegen können.

2. Vorrichtung nach Anspruch 1, **dadurch gekennzeichnet, daß** eine oder beide der Magneteinrichtungen (1, 2) Stabmagnete sind.

3. Vorrichtung nach Anspruch 1, **dadurch gekennzeichnet, daß** eine oder beide Magneteinrichtungen (1, 2) Elektromagnete sind.

4. Vorrichtung nach einem der Ansprüche 1 bis 3, **dadurch gekennzeichnet, daß** eine oder beide Magneteinrichtungen (1, 2) kardanisch aufgehängt sind.

5. Vorrichtung nach einem der Ansprüche 1 bis 4, **dadurch gekennzeichnet, daß** eine oder beide Magneteinrichtungen (1, 2) ausbalanciert sind.

6. Vorrichtung nach Anspruch 5, **dadurch gekennzeichnet, daß** zum Ausbalancieren einer oder beider Magneteinrichtungen (1, 2) ein magnetisches Gegengewicht verwendet wird, vorzugsweise ein der jeweiligen Magneteinrichtung identischer Magnet.

7. Vorrichtung nach einem der Ansprüche 1 bis 6, **dadurch gekennzeichnet, daß** beide Magneteinrichtungen (1, 2) auf einer Halterung (6) angeordnet sind, so daß ihr Abstand und/oder ihre Ausrichtung einstellbar ist.

8. Vorrichtung nach einem der Ansprüche 1 bis 7, **dadurch gekennzeichnet, daß** jede Magneteinrichtung über eine optische und/oder akustische Anzeige (4) verfügt, die auf eine wählbare Nullstellung einstellbar ist.

9. Vorrichtung nach einem der Ansprüche 1 bis 8, **dadurch gekennzeichnet, daß** eine oder beide die Magneteinrichtungen aufnehmenden Halterungen stufenlos arretierbar sind.

## Claims

1. A device for detecting interlocking bores of implants, which has a first magnetic arrangement (1) freely mobile within the magnetic field of a magnet located in the interlocking hole of the implant, **characterised in that** a second, similarly freely mobile magnetic arrangement (2) positioned at a distance from the first magnetic arrangement (1) is provided, wherein the first magnetic arrangement (1) and the second magnetic arrangement (2) are able to move freely within the magnetic field of a magnet provided within the interlocking bore.

2. A device according to Claim 1, **characterised in that** one or both of the magnetic arrangements (1, 2) are bar magnets.

3. A device according to Claim 1, **characterised in that** one or both magnetic arrangements (1, 2) are electromagnets.

4. A device according to one of Claims 1 to 3, **characterised in that** one or both magnetic arrangements (1, 2) are gimbal-mounted.

5. A device according to one of Claims 1 to 4, **characterised in that** one or both of the magnetic arrangements (1, 2) are balanced.

6. A device according to Claim 5, **characterised in that** a magnetic counterweight, preferably a magnet identical to the respective magnetic arrangement, is used to balance one or both magnetic arrangements (1, 2).

7. A device according to one of Claims 1 to 6, **characterised in that** the two magnetic arrangements (1,2) are arranged on a mounting (6), so that their spacing and/or their alignment is adjustable.

8. A device according to one of Claims 1 to 7, **characterised in that** each magnetic arrangement is provided with an optical and/or acoustic indicator (4) which is adjustable to a selectable zero setting.

9. A device according to one of Claims 1 to 8, **characterised in that** one or both of the mountings accommodating the magnetic arrangements are steplessly stoppable.

## Revendications

1. Dispositif pour repérer des trous de fixation d'implants, comprenant un premier dispositif magnétique (1) qui peut se déplacer librement dans le champ magnétique d'un aimant placé dans le trou de fixation de l'implant, **caractérisé en ce qu'**il est prévu un deuxième dispositif magnétique (2) qui est disposé à distance du premier dispositif magnétique (1) et peut également se déplacer librement, le premier dispositif magnétique (1) et le deuxième dispositif magnétique (2) pouvant se déplacer librement dans le champ magnétique d'un aimant placé dans le trou de fixation

2. Dispositif selon la revendication 1, **caractérisé en ce qu'**un ou les deux dispositifs magnétiques (1, 2) sont des barreaux magnétiques.

3. Dispositif selon la revendication 1, **caractérisé en ce qu'**un ou les deux dispositifs magnétiques (1, 2) sont des électroaimants.

4. Dispositif selon une des revendications 1 à 3, **caractérisé en ce qu'**un ou les deux dispositifs magnétiques (1, 2) sont montés sur une liaison à cardan.

5. Dispositif selon une des revendications 1 à 4, **caractérisé en ce qu'**un ou les deux dispositifs magnétiques (1, 2) sont équilibrés.

6. Dispositif selon la revendication 5, **caractérisé en ce que** pour équilibrer un ou les deux dispositifs magnétiques (1, 2), on utilise un contrepoids magnétique, de préférence un aimant identique au dispositif magnétique concerné.

7. Dispositif selon une des revendications 1 à 6, **caractérisé en ce que** les deux dispositifs magnétiques (1, 2) sont montés sur un support (6), de telle sorte que leur espacement et/ou leur orientation sont réglables.

8. Dispositif selon une des revendications 1 à 7, **caractérisé en ce que** chaque dispositif magnétique dispose d'un moyen d'affichage (4) optique et/ou acoustique, qui peut être réglé sur une position zéro sélectionnable.

9. Dispositif selon une des revendications 1 à 8, **caractérisé en ce qu'**un ou les deux supports recevant les dispositifs magnétiques sont positionnables en continu.
